# EUROPÄISCHE PATENTSCHRIFT

(11) **EP 0 736 525 B1**
(45) Veröffentlichungstag und Bekanntmachung des Hinweises auf die Patenterteilung: **12.07.2000**
(21) Anmeldenummer: 96104955.8
(22) Anmeldetag: 28.03.1996
(51) Int. Cl.: C07D 209/14, A61K 31/40

(54) **Benzonitrile als 5-HT Agonisten und Antagonisten**
Benzonitriles as 5-HT agonists and antagonists
Benzonitriles comme 5-HT agonistes et antagonistes

(30) Priorität: 05.04.1995 DE 19512639
(43) Veröffentlichungstag der Anmeldung: 09.10.1996
(73) Patentinhaber: MERCK PATENT GmbH, 64293 Darmstadt (DE)
(72) Erfinder: Böttcher, Henning, Dr., 64287 Darmstadt (DE); Bühring, Karl Ulrich, Dr., 85567 Grafing (DE); Greiner, Hartmut, Dr., 64331 Weiterstadt (DE); Bartoszyk, Gerd, 64331 Weiterstadt (DE); Seyfried, Christoph, Dr., 64342 Seeheim-Jugenheim (DE)

(56) Entgegenhaltungen:
- EP-A- 0 496 222

## Beschreibung

Die Erfindung betrifft 3-[4-(4-(4-Cyan-phenyl)-1-piperazinyl)-butyl]-5-cyan-indol sowie dessen Säureadditionssalze.

Ähnliche Verbindungen sind bekannt aus EP 0 376 607, BE 771285, GB 1 075 156, FR 1.551.082 und insbesondere aus DE 41 01 686 A1 (korrespondierend zu EP 0496 222 A1).
Gegenüber letztgenannter Patentanmeldung zeichnet sich die erfindungsgemäße Verbindung im Vergleich zu den mit Methoxygruppen substituierten bekannten Verbindungen durch eine verbesserte orale Bioverfügbarkeit aus und stellt eine Auswahlerfindung, insbesondere im Hinblick auf DE 41 01 686, dar.

Der Erfindung lag die Aufgabe zugrunde, neue Verbindungen mit wertvollen Eigenschaften aufzufinden, insbesondere solche, die zur Herstellung von Arzneimitteln verwendet werden können. Es wurde gefunden, daß die erfindungsgemäße Verbindung und ihre physiologisch unbedenklichen Säureadditionssalze bei guter Verträglichkeit sehr wertvolle pharmakologische Eigenschaften besitzt. So zeigt sie insbesondere Wirkungen auf das Zentralnervensystem, vor allem 5-HT_{1A}-agonistische und 5-HT-Reuptake hemmende Wirkungen. Sie hemmt die Bindung von tritiierten Serotoninliganden an hippocampale Rezeptoren (Cossery et al., European J. Pharmacol. 140 (1987), 143-155). Außerdem treten Veränderungen der DOPA-Akkumulation im Striatum und der 5-HTP-Akkumulation in N. raphe auf (Seyfried et al., European J. Pharmacol. 160 (1989), 31-41). Weiterhin treten analgetische und blutdrucksenkende Wirkungen auf; so wird bei kathetertragenden wachen, spontan hypertonen Ratten (Stamm SHR/ Okamoto/NIH-MO-CHB-Kisslegg; Methode vgl. Weeks und Jones, Proc. Soc. Exptl. Biol.Med. 104 (1960), 646-648) der direkt gemessene Blutdruck nach peroraler Gabe der Verbindungen gesenkt. Ebenso eignet sie sich zur Prophylaxe und zur Bekämpfung der Folgen cerebraler Infarktgeschehen (apoplexia cerebri) wie Schlaganfall und cerebraler Ischämien, sowie zur Behandlung extrapyramidal-motorischer Nebenwirkungen von Neuroleptika sowie des Morbus Parkinson.

Die erfindungsgemäße Verbindung und ihre physiologisch unbedenklichen Säureadditionssalze kann daher als Arzneimittelwirkstoff insbesondere für Anxiolytika, Antidepressiva, Antipsychotika, Neuroleptika und/oder Antihypertonika und auch als Zwischenprodukte zur Herstellung anderer Arzneimittelwirkstoffe verwendet werden.

Gegenstand der Erfindung ist somit das Arzneimittel 3-[4-(4-(4-Cyan-phenyl)-1-piperazinyl)-butyl]-5-cyan-indol sowie dessen physiologisch unbedenklichen Säureadditionssalze.

Gegenstand der Erfindung sind auch pharmazeutische Zubereitungen, gekennzeichnet durch einen Gehalt an 3-[4-(4-(4-Cyan-phenyl)-1-piperazinyl)-butyl]-5-cyan-indol und/oder einem seiner physiologisch unbedenklichen Säureadditionssalze.

Gegenstand der Erfindung ist auch das Arzneimittel 3-[4-(4-(4-Cyan-phenyl)-1-piperazinyl)-butyl]-5-cyan-indol und seine physiologisch unbedenklichen Säureadditionssalze als 5-Hydroxytryptamin-Agonist und -Antagonist.

Gegenstand der Erfindung ist ferner ein Verfahren zur Herstellung von 3-[4-(4-(4-Cyan-phenyl)-1-piperazinyl)-butyl]-5-cyan-indol nach Anspruch 1 sowie seiner Salze, dadurch gekennzeichnet, daß man
eine Verbindung der Formel II 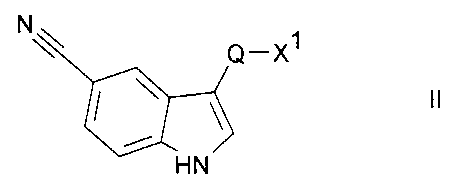 worin
- X¹: X oder NH₂,
- X: Cl, Br, I, OH oder eine reaktionsfähig funktionell abgewandelte OH-Gruppe und
- Q: -(CH₂)₄- bedeutet,
mit einer Verbindung der Formel III

X²-CH₂-CH₂NAr-CH₂-CH₂X³ III

worin
X² und X³ gleich oder verschieden sein können und, falls X¹ = NH₂ ist, jeweils X, andernfalls zusammen NH bedeuten und Ar 4-Cyanphenyl bedeutet,
umsetzt,
oder daß man eine Verbindung der Formel IV 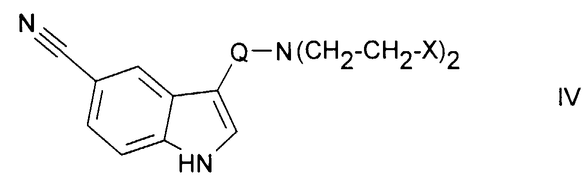 worin
X und Q die angegebenen Bedeutungen haben, mit einer Verbindung der Formel V

Ar-NH₂ V

worin
- Ar: die angegebene Bedeutung hat,
umsetzt,
oder daß man 3-[4-(4-(4-Cyan-phenyl)-1-piperazinyl)-butyl]-5-cyan-indol durch Behandeln mit einer Säure in eines seiner Säureadditionssalze umwandelt.

3-[4-(4-(4-Cyan-phenyl)-1-piperazinyl)-butyl]-5-cyan-indol und auch die Ausgangsstoffe zu seiner Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in DE 4101686) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu 3-[4-(4-(4-Cyan-phenyl)-1-piperazinyl)-butyl]-5-cyan-indol umsetzt. 3-[4-(4-(4-Cyan-phenyl)-1-piperazinyl)-butyl]-5-cyan-indol kann vorzugsweise erhalten werden, indem man Verbindungen der Formel II mit Verbindungen der Formel III umsetzt.

In den Verbindungen der Formel II ist X¹ vorzugsweise X; dementsprechend sind in den Verbindungen der Formel III X² und X³ vorzugsweise zusammen NH. Der Rest X ist vorzugsweise Cl oder Br; er kann jedoch auch I, OH oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy) bedeuten.

Dementsprechend ist 3-[4-(4-(4-Cyan-phenyl)-1-piperazinyl)-butyl]-5-cyan-indol insbesondere durch Umsetzung von Verbindungen der Formel II, worin X¹ Cl oder Br bedeutet, mit Piperazinderivaten der Formel III, worin X² und X³ zusammen eine NH-Gruppe bedeuten (nachstehend als IIIa bezeichnet) erhältlich.

Die Verbindungen der Formeln II und insbesondere III sind zum Teil bekannt; die nicht bekannten Verbindungen der Formeln II und III können leicht analog zu den bekannten Verbindungen hergestellt werden.

Verbindungen der Formel II, worin X¹ OH bedeutet, sind z.B. durch Reduktion der entsprechenden Carbonsäuren oder ihrer Ester erhältlich. Behandeln mit Thionylchlorid, Bromwasserstoff, Phosphortrichlorid oder ähnlichen Halogenverbindungen liefert die entsprechenden Verbindungen der Formel II, worin X¹ Cl oder Br bedeutet. Die entsprechenden Sulfonyloxyverbindungen sind erhältlich aus den Verbindungen der Formel II, worin X¹ OH bedeutet, durch Umsetzung mit den entsprechenden Sulfonsäurechloriden.

Die lodverbindungen der Formel II sind z.B. durch Einwirkung von Kaliumiodid auf die zugehörigen p-Toluolsulfonsäureester erhältlich. Die Verbindungen der Formel II, worin X¹ NH₂ bedeutet, sind z.B. aus den Halogeniden mit Phthalimidkalium herstellbar.

Die Piperazinderivate IIIa sind größtenteils bekannt und z.B. erhältlich durch Umsetzung von Di-(2-chlorethyl)-amin mit dem entsprechenden, am Phenylring substituierten Derivat des Anilins. Verbindungen der Formel III (X² und X³ = jeweils X) sind z.B. herstellbar durch Reduktion von Diestern der Formel AlkylOOC -CH₂-NAr-CH₂- COOAlkyl zu Verbindungen der Formel HO-CH₂-CH₂-NAr-CH₂-CH₂-OH (III, X² = X³ = OH) und gegebenenfalls anschließende Umsetzung mit SOCl₂ bzw. PBr₃.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente der Formel II bzw. des Piperazinderivates der Formel IIIa kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Ferner ist es möglich 3-[4-(4-(4-Cyan-phenyl)-1-piperazinyl)-butyl]-5-cyan-indol zu erhalten, indem man eine Verbindung der Formel IV mit einer Verbindung der Formel V umsetzt.
Die Verbindungen der Formeln IV und insbesondere V sind zum Teil bekannt; die nicht bekannten Verbindungen können leicht in Analogie zu den bekannten hergestellt werden. So lassen sich Verbindungen der Formel IV leicht durch Umsetzung von Verbindungen der Formel II, worin X¹ NH₂ bedeutet, mit 1,2-Dihalogenethan, wobei Halogen bevorzugt für Chlor oder Brom steht, herstellen. Ebenso ist es möglich, Verbindungen des Typs IV durch Umsetzung von Verbindungen der Formel II, worin X¹ Cl, Br oder I bedeutet, mit sekundären Aminen der Formel HN(CH₂-CH₂-X)₂ zu erhalten.

Die primären Amine der Formel V lassen sich ausgehend von Anilin durch die diversen, an sich bekannten Möglichkeiten der elektrophilen Substitution am Aromaten herstellen. Ferner ist es möglich, entsprechend substituierte Nitroverbindungen durch Reduktion in die Amine der Formel V zu überführen.

Die Umsetzung der Verbindungen IV und V verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven, unter Normaldruck oder unter erhöhtem Druck, wobei ein Inertgas wie z.B. N₂ zur Druckerhöhung zugeführt wird. Es ist aber auch möglich, die Verbindungen in Gegenwart eines indifferenten Lösungsmittels umzusetzen. Als Lösungsmittel eignen sich die zuvor bei der Umsetzung von II mit III genannten. Ebenso kann sich der Zusatz eines säurebindenden Mittels zur Reaktionsmischung begünstigend auswirken. Es kommen die gleichen Basen, wie zuvor bei der Umsetzung der Verbindungen II und III beschrieben, in Frage.

Die optimale Reaktionszeit liegt, je nach den gewählten Reaktionsbedingungen, zwischen einigen Minuten und 14 Tagen, die Reaktionstemperatur zwischen etwa 0° und 150°, üblicherweise zwischen 20° und 130°.

3-[4-(4-(4-Cyan-phenyl)-1-piperazinyl)-butyl]-5-cyan-indol kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin- mono- und Disulfonsäuren, Laurylschwefelsäure. Salze mit physiologisch nicht unbedenklichen Säuren, z.B. Pikrate, können zur Isolierung und /oder Aufreinigung von 3-[4-(4-(4-Cyan-phenyl)-1-piperazinyl)-butyl]-5-cyan-indol verwendet werden.

Gegenstand der Erfindung ist weiterhin die Verwendung von 3-[4-(4-(4-Cyan-phenyl)-1-piperazinyl)-butyl]-5-cyan-indol und/oder seiner physiologisch unbedenklichen Salze zur Herstellung pharmazeutischer Zubereitungen, insbesondere auf nicht-chemischem Wege. Hierbei kann es zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

3-[4-(4-(4-Cyan-phenyl)-1-piperazinyl)-butyl]-5-cyan-indol und seine physiologisch unbedenklichen Salze kann bei der therapeutischen Behandlung des menschlichen oder tierischen Körpers und bei der Bekämpfung von Krankheiten verwendet werden. Sie eignet sich zur Behandlung von Erkrankungen des Zentralnervensystems wie Spannungszuständen, Depressionen und/oder Psychosen und von Nebenwirkungen bei der Behandlung der Hypertonie (z.B. mit α-Methyldopa). Ferner kann 3-[4-(4-(4-Cyan-phenyl)-1-piperazinyl)-butyl]-5-cyan-indol in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide und zur Bekämpfung der Folgen cerebraler Infarktgeschehen (Apoplexia cerebri), wie Schlaganfall und cerebraler Ischämien.

Dabei wird die erfindungsgemäße Substanz in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. Im nachfolgenden Beispiel bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Beispiel 1

Man rührt eine Lösung von 2,6 g 3-(4-Chlorbutyl)-5-cyan-indol ("A") und 1,7 g 1-(4-Cyan-phenyl)-piperazin ("B") in 200 ml Acetonitril 12 Stunden bei 20°, arbeitet wie üblich auf und erhält 3-[4-(4-(4-Cyan-phenyl)-1-piperazinyl)-butyl]-5-cyan-indol, Hydrochlorid, F. 262,5-263,5°.

Die nachfolgenden Beispiele betreffen pharmazeutische Zubereitungen:

### Beispiel A: Injektionsgläser

Eine Lösung von 100 g 3-[4-(4-(4-Cyan-phenyl)-1-piperazinyl)-butyl]-5-cyan-indol und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g 3-[4-(4-(4-Cyan-phenyl)-1-piperazinyl)-butyl]-5-cyan-indol mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g 3-[4-(4-(4-Cyan-phenyl)-1-piperazinyl)-butyl]-5-cyan-indol, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg 3-[4-(4-(4-Cyan-phenyl)-1-piperazinyl)-butyl]-5-cyan-indol mit 99,5 g Vaseline unter aseptischen Bedingungen.

### Beispiel E: Tabletten

Ein Gemisch von 1 kg 3-[4-(4-(4-Cyan-phenyl)-1-piperazinyl)-butyl]-5-cyan-indol, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff 3-[4-(4-(4-Cyan-phenyl)-1-piperazinyl)-butyl]-5-cyan-indol werden in üblicher Weise in Hartgelatinekapseln gefüllt, so daß jede Kapsel 20 mg des Wirkstoffs enthält.

### Beispiel H: Ampullen

Eine Lösung von 1 kg 3-[4-(4-(4-Cyan-phenyl)-1-piperazinyl)-butyl]-5-cyan-indol in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. 3-[4-(4-(4-Cyan-phenyl)-1-piperazinyl)-butyl]-5-cyan-indol, sowie dessen Säureadditionssalze.

2. Verfahren zur Herstellung von 3-[4-(4-(4-Cyan-phenyl)-1-piperazinyl)-butyl]-5-cyan-indol nach Anspruch 1 sowie seiner Säureadditionssalze, dadurch gekennzeichnet, daß man
eine Verbindung der Formel II 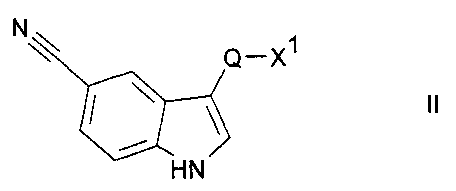 worin
X¹ X oder NH2,
X Cl, Br, I, OH oder eine reaktionsfähig funktionell abgewandelte OH-Gruppe und
Q -(CH₂)₄- bedeutet,
mit einer Verbindung der Formel III
X²-CH₂-CH₂NAr-CH₂-CH₂X³ III
worin
X² und X³ gleich oder verschieden sein können und, falls X¹ = NH₂ ist, jeweils X, andernfalls zusammen NH bedeuten und Ar 4-Cyan-phenyl bedeutet,
umsetzt,
oder daß man eine Verbindung der Formel IV 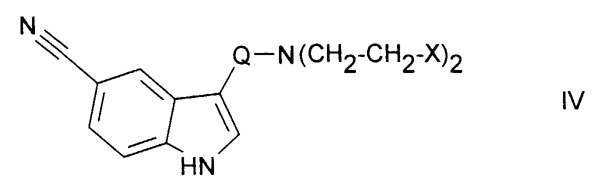 worin
X und Q die angegebenen Bedeutungen haben, mit einer Verbindung der Formel V
Ar-NH₂ V
worin
Ar die angegebene Bedeutung hat,
umsetzt,
oder daß man 3-[4-(4-(4-Cyan-phenyl)-1-piperazinyl)-butyl]-5-cyan-indol durch Behandeln mit einer Säure in eines seiner Säureadditionssalze umwandelt.

3. Das Arzneimittel 3-[4-(4-(4-Cyan-phenyl)-1-piperazinyl)-butyl]-5-cyan-indol nach Anspruch 1 und seine physiologisch unbedenklichen Säureadditionssalze als 5-Hydroxytryptamin-Agonist und -Antagonist.

4. Pharmazeutische Zubereitung, gekennzeichnet durch einen Gehalt an 3-[4-(4-(4-Cyan-phenyl)-1-piperazinyl)-butyl]-5-cyan-indol nach Anspruch 1 und/oder einem seiner physiologisch unbedenklichen Säureadditionssalze.

5. Verfahren zur Herstellung pharmazeutischer Zubereitungen, dadurch gekennzeichnet, daß man 3-[4-(4-(4-Cyan-phenyl)-1-piperazinyl)-butyl]-5-cyan-indol nach Anspruch 1 und/oder eines seiner physiologisch unbedenklichen Säureadditionssalze zusammen mit mindestens einem festen, flüssigen oder halbflüssigen Träger- oder Hilfsstoff in eine geeignete Dosierungsform bringt.

6. Verwendung von 3-[4-(4-(4-Cyan-phenyl)-1-piperazinyl)-butyl]-5-cyan-indol nach Anspruch 1 und/oder seiner physiologisch unbedenklichen Säureadditionssalze zur Herstellung eines Arzneimittels.

## Claims

1. 3-[4-(4-(4-Cyanophenyl)-1-piperazinyl)butyl]-5-cyanoindole and its acid addition salts.

2. Process for the preparation of 3-[4-(4-(4-cyanophenyl)-1-piperazinyl)butyl]-5-cyanoindole according to Claim 1, and its acid addition salts, characterized in that
a compound of the formula II 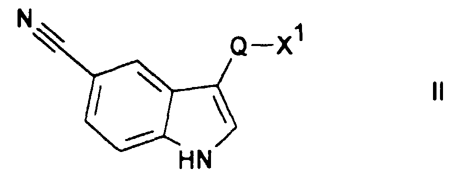 in which
X1 is X or NH₂,
X is Cl, Br, I, OH or a reactively [sic] functionally modified OH group, and
Q is -(CH₂)₄-,
is reacted with a compound of the formula III
X²-CH₂-CH₂NAr-CH₂-CH₂-X³ III
in which
X² and X³ can be identical or different and, if X¹ = NH₂, are each X, or else together NH and Ar is 4-cyanophenyl,
or in that a compound of the formula IV 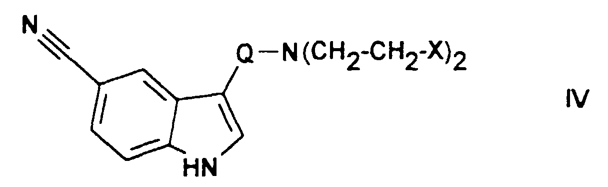 in which
X and Q have the meanings indicated, is reacted with a compound of the formula V
Ar-NH2 V
in which
Ar has the meaning indicated,
or in that 3-[4-(4-(4-cyanophenyl)-1-piperazinyl)-butyl]-5-cyanoindole is converted into one of its acid addition salts by treating with an acid.

3. The medicament 3-[4-(4-(4-cyanophenyl)-1-piperazinyl)butyl]-5-cyanoindole according to Claim 1 and its physiologically acceptable acid addition salts as a 5-hydroxytryptamine agonist and antagonist.

4. Pharmaceutical preparation, characterized in that it contains 3-[4-(4-(4-cyanophenyl)-1-piperazinyl)butyl]-5-cyanoindole according to Claim 1 and/or one of its physiologically acceptable acid addition salts.

5. Process for the production of pharmaceutical preparations, characterized in that 3-[4-(4-(4-cycanophenyl)-1-piperazinyl)butyl]-5-cyanoindole according to Claim 1 and/or one of its physiologically acceptable acid addition salts is brought into a suitable dose form together with at least one solid, liquid or semiliquid excipient or auxiliary.

6. Use of 3- [4- (4- (4-cycanophenyl)-1-piperazinyl)-butyl]-5-cyanoindole according to Claim 1 and/or its physiologically acceptable acid addition salts for the production of a medicament.

## Revendications

1. Le 3-[4-(4-(4-cyanophényl)-1-pipérazinyl)-butyl]-5-cyanoindol, ainsi que ses sels d'addition d'acides.

2. Procédé pour la préparation du 3-[4-(4-(4-cyanophényl)-1-pipérazinyl)-butyl] -5-cyanoindol selon la revendication 1, ainsi que de ses sels d'addition d'acides, caractérisé en ce que l'on fait réagir un composé de 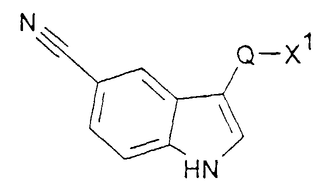 formule II :
où
X¹ représente X ou NH₂,
X représente Cl, Br, I, OH ou un groupe OH réactif, modifié fonctionnellement, et II
Q représente -(CH₂)₄-
avec un composé de formule III :
X²-CH₂-CH₂NAr-CH₂-CH₂X³ III
où
X² et X³ peuvent être identiques ou différents et représentent, dans le cas où X¹ est NH₂, X, sinon ils représentent ensemble NH et Ar représente le 4-cyanophényle,
ou en ce que l'on fait réagir un composé de formule IV : 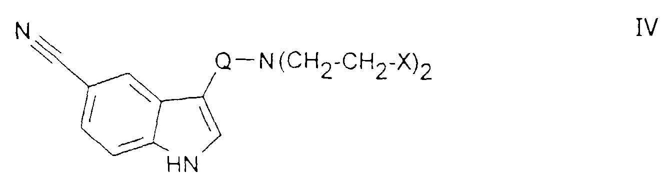 où
X et Q ont les significations indiquées, avec un composé de formule V
Ar-NH₂ V
où
Ar a la signification indiquée,
ou en ce que l'on transforme le 3-[4-(4-(4-cyanophényl)-1-pipérazinyl)-butyl]-5-cyanoindol par traitement avec un acide en l'un de ses sels d'addition d'acides.

3. Le médicament 3-[4-(4-(4-cyanophényl)-1-pipérazinyl)-butyl]-5-cyanoindol selon la revendication 1 et ses sels d'addition d'acides physiologiquement acceptables comme agoniste et antagoniste de la 5-hydroxytryptamine.

4. Préparation pharmaceutique caractérisée en ce qu'elle contient du 3-[4-(4-(4-cyanophényl)-1-pipérazinyl)-butyl]-5-cyanoindol selon la revendication 1 et/ou l'un de ses sels d'addition d'acides physiologiquement acceptables.

5. Procédé pour la fabrication de préparations pharmaceutiques, caractérisé en ce que l'on met sous une forme de dosage appropriée le 3-[4-(4-(4-cyano-phényl)-1-pipérazinyl)-butyl]-5-cyanoindol selon la revendication 1 et/ou l'un de ses sels d'addition d'acides physiologiquement acceptables, associé à au moins un support ou un adjuvant solide, liquide ou semi-liquide.

6. Utilisation du 3-[4-(4-(4-cyanophényl)-1-pipérazinyl)-butyl]-5-cyanoindol selon la revendication 1 et/ou de ses sels d'addition d'acides physiologiquement acceptables pour la fabrication d'un médicament.
